# EUROPEAN PATENT SPECIFICATION

(11) **EP 2 578 208 B1**
(45) Date of publication and mention of the grant of the patent: **21.05.2014**
(21) Application number: 12187333.5
(22) Date of filing: 05.10.2012
(51) Int. Cl.: A61K 9/16, A61K 9/20

(54) **DPP-IV inhibitor solid dosage formulations**
Feste Dosierformulierungen von DPP-IV Inhibitoren
Formulations posologiques solides d'inhibiteurs DPP-IV

(30) Priority: 06.10.2011 TR 201109893
(43) Date of publication of application: 10.04.2013
(73) Proprietor: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Saydam, Mehtap, 34460 Istanbul (TR); Yelken, Gülay, 34460 Istanbul (TR)
(74) Representative: Sevinç, Erkan

(56) References cited:
- WO-A2-2006/078593
- WO-A2-2007/096906

## Description

### Technical Field

The present invention is relates to formulation comprising DPP-IV inhibitor. More specifically, this invention is related to a stable formulation of vildagliptin having content uniformity, dissolution rate and solubility values at the desired levels.

### Background of the Invention

Diabetes may develop depending on many factors. In the glucose tolerance test performed, characteristically high levels of plasma and hyperglycemia are observed after the oral administration of glucose. Type 2 diabetes patients carry the risk of macro vascular and micro vascular complications including especially coronary heart disease, paralysis, peripheral vascular disease, hypertension, nephropathy, neuropathy and retinopathy. For the reasons mentioned above, it is vital to get the diabetes under control.

DPP-IV is a serine protease bound to membrane and found in the plasma in a soluble form. It is found in several tissues including vascular endothelial cells and immunity system cells (CD26, which is an indicator of active T cells). Nevertheless, the specific DPP-IV inhibition did not effect the CD26 immune activation. The activation and proliferation of T-cells were found to be related to DPP-8 and DPP-9. The increase in the levels of endogenous GLP-1 and GIP in connection with the development of oral selective DPP-IV inhibitors, provides the formation of anti-diabetic effect via the insulin and glucagon depending on the physiological glucose.

Vildagliptin is a dipeptidyl dipeptidase-IV (DPP-IV) inhibitor developed for the treatment of type 2 diabetes (insulin independent diabetes). By inhibiting the degradation of dipeptidyl dipeptidase-IV enzyme, vildagliptin inhibits the effects of the incretin hormones, glucagon-like peptide-1 (GLP-1) and glucose dependant insulinotropic peptide (GIP). The chemical name of the vildagliptine is S)-{[(3-Hydroxyadamantan-1-yl)amino]acetyl}pyrrolidine-2-carbonitrile and its chemical structure is shown in Formula 1.

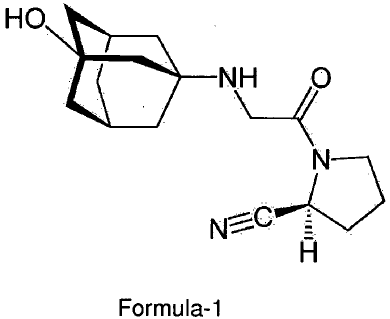

Vildagliptin dissolves in water and in organic polar solvents.

It is commercially available in the dosage amount of 50 mg under the brand name vildagliptin Galvus®. It is used in diabetes, particularly in the treatment of type 2 diabetes.

There are various patents related to vildagliptine in the patent literature.

In patent application WO0034241, vildagliptin or its acid added salt and their usage in diabetes and obesity is described.

With the patent application WO2006078593, DPP-IV and preferably the formulation comprising vildagliptin or its acid added salt obtained by direct compression is protected.

With the patent application no WO2006135723, a formulation comprising preferably vildagliptin as active agent with hydroxy propyl methyl cellulose, microcrystal cellulose and magnesium stearate is disclosed.

Saxagliptin is a dipeptidyl dipeptidase-IV (DPP-IV) inhibitor, which is developed for the treatment of type 2 diabetes (insulin independent diabetes).

The chemical name of the vildagliptine is (2S,4S,5R)-2-[(2S)-2-Amino-2-(3-hydroxyadamantan-1-yl)acetyl]-2-azabicyclo[3.1.0]hexane-3-carbonitrile and its chemical structure is shown in Formula 2.

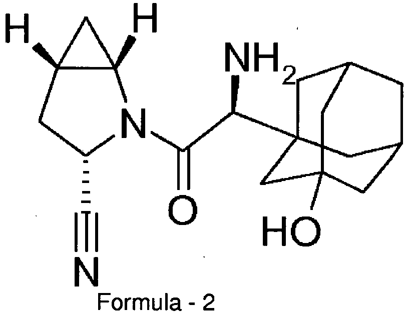

It is commercially available in the dosage amounts of 2.5 or 5 mg, under the brand name Saxagliptin ONGLYZA®. It is used in diabetes, particularly in the treatment of type 2 diabetes.

There are various patents related to saxagliptin in the patent literature.

Saxagliptin and its application in the treatment are described in patent application US6395767.

In patent application W02010115974, anhydrous crystal of the saxagliptin hydrochloride containing not more than 1.5 % water by weight is described.

With the patent application WO2005115982, the process related to the production of the saxagliptin is explained.

With the patent application WO2005117841, the pharmaceutical formulation of saxagliptin which contains inner and outer coatings and the process for obtaining this formulation are explained.

Sitagliptin is a dipeptidyl dipeptidase-IV (DPP-IV) inhibitor, which is developed for the treatment of type 2 diabetes (insulin independent diabetes). By inhibiting the degradation of dipeptidyl dipeptidase-4 enzyme, sitagliptin inhibits the effects of incretin hormones, glucagon-like peptide-1 (GLP-1) and glucose dependant insulinotropic peptide (GIP). The chemical name of the sitagliptin is (R)-3-Amino-1-[3-(triflourmethyl)-5,6,7,8-tetrahydro [1,2,4]triazole[4,3-a]pirazin-7-yl]-4-(2,4,5-triflourphenyl)butan-1-on and its chemical structure is shown in Formula 3.

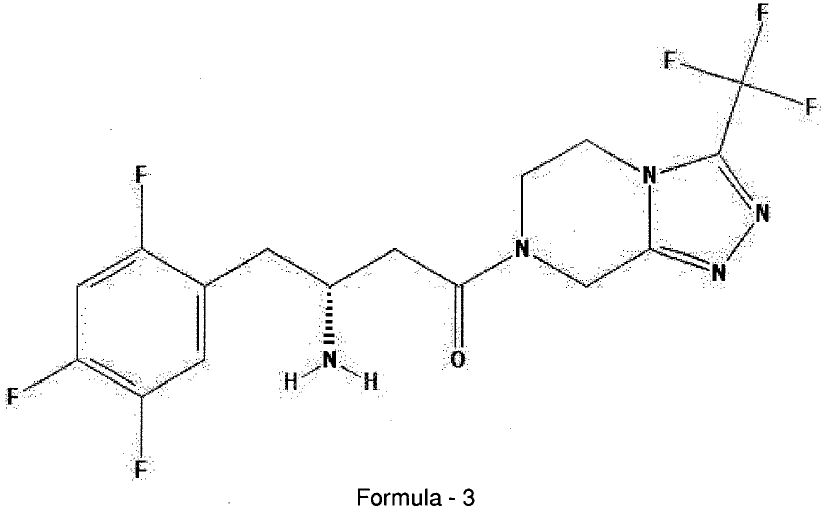

It is commercially available in the dosage amounts of 25, 50 or 100 mg, under the brand name Sitaglipitin Januvia®. It is used in diabetes, particularly in the treatment of type 2 diabetes.

Sitagliptin is described in the patent US6699871. With the patent WO2005003135, sitagliptin crystal phosphate monohydrate is explained.

The solubility and dissolution rate of vildagliptin, sitagliptin and saxagliptin directly affect the bioavailability. Therefore, it is very important to increase the solubility and dissolution rate of the mentioned active ingredients.

Another problem related to these active ingredients is the stability problem experienced due to environmental and physical conditions as it is also the case in many other active ingredients. DPP-IV inhibitors are highly affected from the temperature, air and humidity conditions as well as the solvents used during their formulation. Exposure to air and humidity causes the structural degradation of the said active ingredients and changes their chemical behaviours. The stability of the developed products is not at a desired level and their shelf life shortened. Additionally, these active ingredients may react with the excipients used at the development step of the formulation. This situation forms impurities in the formulation and causes the incorporation of unwanted compositions into the formulation.

The difference in the particle sizes of the active ingredients prevents the ingredient uniformity of the formulation. This is an undesired situation for the user.

Additionally, another problem experienced in the development of the present formulation with the said active ingredients is the fluidity problem which complicates the production. Also, during the manufacturing process stage, these substances may stick to the puncher and to the surface of the other equipments used.

By virtue of the said disadvantages and needs, there exists a need for the improvement of the technical field related to the DPP-IV formulations having anti-diabetic effect.

### Objective and Brief Description of the Invention

The present invention is an easily applicable DPP-IV inhibitor formulation which eliminates all of the problems mentioned above and provides additional advantages for the related technical field.

Based on the state of the art, the main objective of the invention is providing at least one stable formulation having anti-diabetic activity.

Another objective of the invention is obtaining a formulation having content uniformity.

Another objective of the invention is realizing a formulation with the desired solubility level and dissolution rate and consequently, a formulation with the desired level of bioavailability.

Another objective of the invention is to prevent the active ingredient from being affected from its solvent by not using any liquid solvent including water.

Another objective of the invention is to diminish the energy needed for manufacturing.

Another objective of the invention is to develop a formulation which does not exhibit fluidity problems during manufacture.

In order to achieve all the goals which are mentioned above and which may be revealed from the detailed description below, a solid dosage formulation obtained by briquette pressing or by compression between rollers is developed.

In a preferred embodiment of the invention, the said novelty is realized by the DPP-IV inhibitor and at least one binding dry granulation excipient.

In another preferred embodiment of the present invention, the formulation in question does not comprise a liquid solvent.

In a preferred embodiment of the invention, the said DPP-IV inhibitor is at least one of the substances selected from the group containing vildagliptin, saxagliptin and sitagliptin.

In a preferred embodiment of the present invention the said DPP-IV inhibitor is vildagliptin.

In another preferred embodiment of the present invention, the said binding dry granulation excipient is polycarbophil.

A preferred embodiment of the invention additionally contains at least one binding dry granulation excipient selected from the group consisting of calcium carbonate, microcrystalline cellulose, silicified microcrystalline cellulose, low viscosity hydroxy propyl cellulose (fine particle), low viscosity hydroxy propyl methyl cellulose, low viscosity ethyl cellulose, low viscosity methyl cellulose, polyethylene glycol, carbomer, methacrylic acid copolymer, amino methacrylate copolymere, beeswax, mixture of solid maltodextrin-corn syrup, corn starch, hydrolyzed gelatine, polyvinyl acetate, polyvinyl pyrrolidone, polyvinyl pyrrolidone-vinyl acetate copolymer, sugars, glucose syrup, natural gums (glisirhiza gum, guar gum, tragagant gum), collagen, agar, alginate, sodium alginate, carrageenans, hyaluronic acid, pectin, poloxamer, polyacrylamide, polyvinyl alcohol, aluminium hydroxide, bentonite, laponit, setostearyl alcohol, polyoxyethylene-alkyl ethers, acacia mucilage, polydextrose, polyethylene oxide or a mixture of them.

In a preferred embodiment of the invention, the ratio interval of the said vildagliptin to polycarbophil is 1 -100, preferably 6 - 80, and more preferably 10 - 50.

In a preferred embodiment of it, the invention also comprises one or more excipients.

In a preferred embodiment of the invention, the said excipient comprises at least one of diluents, disintegrants, glidants and lubricants or a mixture of them in a suitable amount.

In a preferred embodiment of it, the invention comprises at least one diluent selected from the group comprising lactose, starch, calcium carbonate, microcrystalline cellulose, silicified microcrystalline cellulose, mannitol, sorbitol, sucrose, dextrose, dextrates, lactitol, maltodextrine, xylitol, trehalose, dibasic calcium phosphate anhydrate and dibasic calcium phosphate dihydrate or a mixture of them in a suitable amount.
In a preferred embodiment of the invention, the ratio of silicified microcrystalline cellulose to dibasic calcium phosphate anhydrate is 3/1.

In a preferred embodiment of the invention, the said disintegrant is croscarmellose sodium or sodium starch glycolate or a mixture of them in suitable amounts.

In a preferred embodiment of the present invention the said glidant is colloidal silicone dioxide.

In a preferred embodiment of it, the invention preferably comprises polyethylene glycol or magnesium stearate or a mixture of them in a suitable amount as lubricant.

In a preferred embodiment of the invention, at least a part of the said lubricant or lubricant mixture is used in the inner granule phase and the rest of it is used in the outer granule phase.

In a preferred embodiment of the invention, the average particle size of the granules obtained by briquette compression or pressing between rollers is between 25- 1250 µm, preferably between 75- 800 µm and more preferably between 100- 600 µm.

In another preferred embodiment of the present invention, the said formulation is in the form of tablet or capsule.

In another preferred embodiment of the present invention, the said tablet hardness is 20-300 N, preferably 30 - 250 N, and more preferably 50 - 100 N.

In another preferred embodiment of the present invention, the compression force of the formulation dry granulation process is 1-25 KN, preferably 3 - 18 KN, and more preferably 5 - 10 KN.

In a preferred embodiment of the present invention, the said pharmaceutical formulation consist of,
a. 5-60 % vildagliptin or a pharmaceutically acceptable salt of it by weight
b. 0.1-30 % polycarbophyl by weight
c. 5-90 % silicified microcrystalline cellulose by weight
d. 0.25-20 % croscarmellose sodium by weight
e. 0.1-1 % colloidal silicone dioxide by weight
f. 0.1-3 % magnesium stearate by weight

In a preferred embodiment of the present invention, the said pharmaceutical formulation consist of,
a. 5-60 % vildagliptin or a pharmaceutically acceptable salt of it by weight,
b. 0.1-30 % polycarbophil by weight
c. 2-40 % copolymer of vinyl pyrrolidone-vinyl acetate or low viscosity hydroxy propyl cellulose by weight
d. 5-90 % silicified microcrystalline cellulose by weight
e. 0.25-20 % croscarmellose sodium by weight
f. 0.1-1 % colloidal silicone dioxide by weight
g. 0.1-3 % magnesium stearate by weight

In a preferred embodiment of the present invention, the said pharmaceutical formulation consist of,
a. 5-60 % vildagliptin or a pharmaceutically acceptable salt of it by weight,
b. 0.5-30 % low viscosity hydroxy propyl cellulose by weight
c. 5-90 % silicified microcrystalline cellulose by weight
d. 5-90 % dibasic calcium phosphate anhydrate by weight
e. 0.25-20 % croscarmellose sodium by weight
f. 0.1-1 % colloidal silicone dioxide by weight
g. 0.1-3 % magnesium stearate by weight

Another preferred embodiment of the present invention is a method for preparing the said pharmaceutical formulation, comprasing the steps below
a. Mixing vildagliptin, polycarbophyl and silicified microcrystalline cellulose,
b. then, granulating the mixture in a roller compactor or making briquette press and granulating it by sieving it with a suitable sieve,
c. adding croscarmellose sodium and colloidal silicone dioxide to the obtained granule by sieving and mixing them,
d. adding magnesium stearate afterwards to this mixture by sieving and mixing for a short time,
e. tablet compressing with the obtained powder ready for compression or filling it into a capsule,
f. coating the tablet with polyvinyl alcohol or polyvinyl alcohol polyethylene glycol graft copolymer.

Another preferred embodiment of the present invention is a method for preparing the said pharmaceutical formulation, comprasing the steps below:
a. mixing vildagliptin, polycarbophyl, vinyl pyrrolidone-vinyl acetate copolymer or low viscosity hydroxy propyl cellulose and silicified microcrystalline cellulose,
b. granulating then the mixture in a roller compactor or making briquette press and granulating it by sieving with a suitable sieve,
c. adding croscarmellose sodium and colloidal silicone dioxide to the obtained granule by sieving and mixing them,
d. afterwards, adding magnesium stearate to this mixture by sieving and mixing for a short time,
e. tablet compressing with the obtained powder ready for compression or filling it into a capsule,
f. coating the tablet with polyvinyl alcohol or polyvinyl alcohol polyethylene glycol graft copolymer

Another preferred embodiment of the present invention is a method for preparing the said pharmaceutical formulation comprasing the steps below:
a. mixing vildagliptin, binder and diluent,
b. afterwards, adding at least a fragment of magnesium stearate to this mixture and mixing them,
c. granulating then the mixture in a roller compactor or making briquette press and granulating it by sieving with a suitable sieve,
d. adding croscarmellose sodium and colloidal silicone dioxide to the obtained granule by sieving and mixing them,
e. afterwards, adding the rest of the magnesium stearate to this mixture by sieving and mixing for a short time,
f. tablet compressing with the obtained powder ready for compression or filling it into a capsule,
g. coating the tablet with polyvinyl alcohol or polyvinyl alcohol polyethylene glycol graft copolymer

### Detailed Description of the Invention

### Example-1 Capsule or Tablet

| **Ingredients** | **amount % (mg)** |
|---|---|
| vildagliptin | 5 - 60% |
| polycarbophil | 0,1 - 30 % |
| silicified microcrystalline cellulose | 5 - 90% |
| croscarmellose sodium | 0,25 - 20 % |
| colloidal silicone dioxide | 0,1 - 1 % |
| magnesium stearate | 0,1 - 3 % |

The production of the formulation is realized like this. Vildagliptin, polycarbophil and silicified microcrystalline cellulose are mixed and granulated in roller compactor or briquette pressed. Then, the mixture is granulated by sieving with an appropriate screen. To the obtained granule, croscarmellose sodium and colloidal silicone dioxide are added after being sieved and they are mixed. Afterwards, magnesium stearate is added after being sieved and mixed for a short time. The tablet is compressed with the obtained powder ready for compression or the capsule is filled. The coating process is realized with a coating material having humidity barrier such as Opadry AMB/ Kollicoat IR.

In another production method, vildagliptin is mixed with polycarbophil and silicified microcrystalline cellulose. Half of the magnesium stearate is added to this mixture by sieving. After mixing for a short time, the mixture is mangled from the roller compactor or briquette pressed and granulated by sieving. To the obtained granules, croscarmellose sodium and colloidal silicone dioxide are added and mixed. The rest of the magnesium stearate is added to the this mixture by sieving and after mixing for a short time the tablet compressing and capsule filling processes are realized. The coating process is realized with a coating material having humidity barrier such as Opadry AMB/ Kollicoat IR.

### Example-2 capsule or tablet

| **Ingredients** | **amount % (mg)** |
|---|---|
| vildagliptin | 5 - 60 % |
| polycarbophil | 0,1 - 30 % |
| vinyl pyrrolidone-vinyl acetate copolymer or low viscosity hydroxy propyl cellulose | 2 - 40 % |
| silicified microcrystalline cellulose or dibasic calcium phosphate anhydrate | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized as below. Vildagliptin, polycarbophil, vinyl pyrrolidone-vinyl acetate copolymer or low viscosity hydroxy propyl cellulose and silicified microcrystalline cellulose or dibasic calcium phosphate anhydrate are mixed. The mixture is granulated in roller compactor or briquette pressed and granulated by sieving through the suitable sieve. To the obtained granule, croscarmellose sodium and colloidal silicone dioxide are added by sieving and they are mixed. Afterwards, magnesium stearate is added by sieving and mixed for a short time. The tablet is compressed with the obtained powder ready for compression or the capsule is filled. The coating process is realized with a coating material having humidity barrier such as Opadry AMB/ Kollicoat IR.

In another production method, vildagliptin is mixed with polycarbophyl, low viscosity hydroxy propyl cellulose and dibasic calcium phosphate anhydrate. Half of the magnesium stearate is sieved and added to the powder mixture and after mixing for a short time, the mixture is mangled from the roller compactor or slug pressed and granulated by sieving. Croscarmellose sodium and colloidal silicone dioxide are added to the obtained granules and mixed. The rest of the magnesium stearate is added to the this mixture by sieving and mixed for a short time and the tablet pressing process and capsule filling are realized. The coating process is realized with a coating material having humidity barrier such as Opadry AMB/ Kollicoat IR.

### Example-3 capsule or tablet

| Ingredients | amount % (mg) |
|---|---|
| vildagliptin | 5 - 60 % |
| low viscosity hydroxy propyl cellulose | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, low viscosity hydroxy propyl cellulose and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with polycarbophil, low viscosity hydroxy propyl methyl cellulose, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-4 Capsule or Tablet

| **Ingredients** | **amount % (mg)** |
|---|---|
| vildagliptin | 5 - 60 % |
| methyl cellulose | 0,1 - 30 % |
| silicified microcrystalline cellulose | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized like this. Vildagliptin, methyl cellulose and silicified microcrystalline cellulose are mixed and granulated in roller compactor or briquette pressed. Then, the mixture is granulated by sieving with an appropriate screen. To the obtained granule, croscarmellose sodium and colloidal silicone dioxide are added after being sieved and they are mixed. Afterwards, magnesium stearate is added after being sieved and mixed for a short time. The tablet is compressed with the obtained powder ready for compression or the capsule is filled. The coating process is realized with a coating material having humidity barrier such as Opadry AMB/ Kollicoat IR.

In another production method, vildagliptin is mixed with methyl cellulose and silicified microcrystalline cellulose. Half of the magnesium stearate is added to this mixture by sieving. After mixing for a short time, the mixture is mangled from the roller compactor or briquette pressed and granulated by sieving. To the obtained granules, croscarmellose sodium and colloidal silicone dioxide are added and mixed. The rest of the magnesium stearate is added to the this mixture by sieving and after mixing for a short time the tablet compressing and capsule filling processes are realized. The coating process is realized with a coating material having humidity barrier such as Opadry AMB/ Kollicoat IR.

### Example-5 capsule or tablet

| **Ingredients** | **amount % (mg)** |
|---|---|
| vildagliptin | 5 - 60 % |
| polycarbophil | 0,1 - 30 % |
| methyl cellulose | 2 - 40 % |
| silicified microcrystalline cellulose or dibasic calcium phosphate anhydrate | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized as below. Vildagliptin, polycarbophil, methyl cellulose and silicified microcrystalline cellulose or dibasic calcium phosphate anhydrate are mixed. The mixture is granulated in roller compactor or briquette pressed and granulated by sieving through the suitable sieve. To the obtained granule, croscarmellose sodium and colloidal silicone dioxide are added by sieving and they are mixed. Afterwards, magnesium stearate is added by sieving and mixed for a short time. The tablet is compressed with the obtained powder ready for compression or the capsule is filled. The coating process is realized with a coating material having humidity barrier such as Opadry AMB/ Kollicoat IR.

In another production method, vildagliptin is mixed with polycarbophyl, methyl cellulose and dibasic calcium phosphate anhydrate. Half of the magnesium stearate is sieved and added to the powder mixture and after mixing for a short time, the mixture is mangled from the roller compactor or slug pressed and granulated by sieving. Croscarmellose sodium and colloidal silicone dioxide are added to the obtained granules and mixed. The rest of the magnesium stearate is added to the this mixture by sieving and mixed for a short time and the tablet pressing process and capsule filling are realized. The coating process is realized with a coating material having humidity barrier such as Opadry AMB/ Kollicoat IR.

### Example-6 capsule or tablet

| Ingredients | amount % (mg) |
|---|---|
| vildagliptin | 5 - 60 % |
| polyethylene oxide | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, polyethylene oxide and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with polyethylene oxide, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-7 capsule or tablet

| Ingredients | amount % (mg) |
|---|---|
| vildagliptin | 5 - 60 % |
| carbomer | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, carbomer and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with carbomer, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-8 capsule or tablet

| Ingredients | amount % (mg) |
|---|---|
| vildagliptin | 5 - 60 % |
| polyethylene glycol | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, polyethylene glycol and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with polyethylene glycol, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-9 capsule or tablet

| Ingredients | amount % (mg) |
|---|---|
| vildagliptin | 5 - 60 % |
| Sodium alginate and/or alginic acid | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, sodium alginate and/or alginic acid and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with sodium alginate and/or alginic acid, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-10 capsule or tablet

| Ingredients | amount % (mg) |
|---|---|
| vildagliptin | 5 - 60 % |
| polyvinyl alcohol | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, polyvinyl alcohol and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with polyvinyl alcohol, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-11 capsule or tablet

| Ingredients | amount % (mg) |
|---|---|
| vildagliptin | 5 - 60 % |
| poloxamer | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Vildagliptin, poloxamer and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, vildagliptin is mixed with poloxamer, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-12 Capsule or Tablet

| **Ingredients** | **amount % (mg)** |
|---|---|
| saxagliptin | 0,2 - 20 % |
| polycarbophil | 0,1 - 30 % |
| silicified microcrystalline cellulose | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized like this. Saxagliptin, polycarbophil and silicified microcrystalline cellulose are mixed and granulated in roller compactor or briquette pressed. Then, the mixture is granulated by sieving with an appropriate screen. To the obtained granule, croscarmellose sodium and colloidal silicone dioxide are added after being sieved and they are mixed. Afterwards, magnesium stearate is added after being sieved and mixed for a short time. The tablet is compressed with the obtained powder ready for compression or the capsule is filled. The coating process is realized with a coating material having humidity barrier such as Opadry AMB/ Kollicoat IR.

In another production method, saxagliptin is mixed with polycarbophil and silicified microcrystalline cellulose. Half of the magnesium stearate is added to this mixture by sieving. After mixing for a short time, the mixture is mangled from the roller compactor or briquette pressed and granulated by sieving. To the obtained granules, croscarmellose sodium and colloidal silicone dioxide are added and mixed. The rest of the magnesium stearate is added to the this mixture by sieving and after mixing for a short time the tablet compressing and capsule filling processes are realized. The coating process is realized with a coating material having humidity barrier such as Opadry AMB/ Kollicoat IR.

### Example-13 capsule or tablet

| **Ingredients** | **amount % (mg)** |
|---|---|
| saxagliptin | 0,2 - 20 % |
| polycarbophil | 0,1 - 30 % |
| vinyl pyrrolidone-vinyl acetate copolymer or low viscosity hydroxy propyl cellulose | 2 - 40 % |
| silicified microcrystalline cellulose or dibasic calcium phosphate anhydrate | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized as below. Saxagliptin, polycarbophil, vinyl pyrrolidone-vinyl acetate copolymer or low viscosity hydroxy propyl cellulose and silicified microcrystalline cellulose or dibasic calcium phosphate anhydrate are mixed. The mixture is granulated in roller compactor or briquette pressed and granulated by sieving through the suitable sieve. To the obtained granule, croscarmellose sodium and colloidal silicone dioxide are added by sieving and they are mixed. Afterwards, magnesium stearate is added by sieving and mixed for a short time. The tablet is compressed with the obtained powder ready for compression or the capsule is filled. The coating process is realized with a coating material having humidity barrier such as Opadry AMB/ Kollicoat IR.

In another production method, saxagliptin is mixed with polycarbophyl, low viscosity hydroxy propyl cellulose and dibasic calcium phosphate anhydrate. Half of the magnesium stearate is sieved and added to the powder mixture and after mixing for a short time, the mixture is mangled from the roller compactor or slug pressed and granulated by sieving. Croscarmellose sodium and colloidal silicone dioxide are added to the obtained granules and mixed. The rest of the magnesium stearate is added to the this mixture by sieving and mixed for a short time and the tablet pressing process and capsule filling are realized. The coating process is realized with a coating material having humidity barrier such as Opadry AMB/ Kollicoat IR.

### Example-14 capsule or tablet

| Ingredients | amount % (mg) |
|---|---|
| saxagliptin | 0,2 - 20 % |
| low viscosity hydroxy propyl cellulose | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Saxagliptin, low viscosity hydroxy propyl cellulose and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with polycarbophil, low viscosity hydroxy propyl methyl cellulose, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-15 Capsule or Tablet

| **Ingredients** | **amount % (mg)** |
|---|---|
| saxagliptin | 0,2 - 20 % |
| methyl cellulose | 0,1 - 30 % |
| silicified microcrystalline cellulose | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized like this. Saxagliptin, methyl cellulose and silicified microcrystalline cellulose are mixed and granulated in roller compactor or briquette pressed. Then, the mixture is granulated by sieving with an appropriate screen. To the obtained granule, croscarmellose sodium and colloidal silicone dioxide are added after being sieved and they are mixed. Afterwards, magnesium stearate is added after being sieved and mixed for a short time. The tablet is compressed with the obtained powder ready for compression or the capsule is filled. The coating process is realized with a coating material having humidity barrier such as Opadry AMB/ Kollicoat IR.

In another production method, saxagliptin is mixed with methyl cellulose and silicified microcrystalline cellulose. Half of the magnesium stearate is added to this mixture by sieving. After mixing for a short time, the mixture is mangled from the roller compactor or briquette pressed and granulated by sieving. To the obtained granules, croscarmellose sodium and colloidal silicone dioxide are added and mixed. The rest of the magnesium stearate is added to the this mixture by sieving and after mixing for a short time the tablet compressing and capsule filling processes are realized. The coating process is realized with a coating material having humidity barrier such as Opadry AMB/ Kollicoat IR.

### Example-16 capsule or tablet

| **Ingredients** | **amount % (mg)** |
|---|---|
| saxagliptin | 0,2 - 20 % |
| polycarbophil | 0,1 - 30 % |
| methyl cellulose | 2 - 40 % |
| silicified microcrystalline cellulose or dibasic calcium phosphate anhydrate | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized as below. Saxagliptin, polycarbophil, methyl cellulose and silicified microcrystalline cellulose or dibasic calcium phosphate anhydrate are mixed. The mixture is granulated in roller compactor or briquette pressed and granulated by sieving through the suitable sieve. To the obtained granule, croscarmellose sodium and colloidal silicone dioxide are added by sieving and they are mixed. Afterwards, magnesium stearate is added by sieving and mixed for a short time. The tablet is compressed with the obtained powder ready for compression or the capsule is filled. The coating process is realized with a coating material having humidity barrier such as Opadry AMB/ Kollicoat IR.

In another production method, Saxagliptin is mixed with polycarbophyl, methyl cellulose and dibasic calcium phosphate anhydrate. Half of the magnesium stearate is sieved and added to the powder mixture and after mixing for a short time, the mixture is mangled from the roller compactor or slug pressed and granulated by sieving. Croscarmellose sodium and colloidal silicone dioxide are added to the obtained granules and mixed. The rest of the magnesium stearate is added to the this mixture by sieving and mixed for a short time and the tablet pressing process and capsule filling are realized. The coating process is realized with a coating material having humidity barrier such as Opadry AMB/ Kollicoat IR.

### Example-17 capsule or tablet

| Ingredients | amount % (mg) |
|---|---|
| saxagliptin | 0,2 - 20 % |
| polyethylene oxide | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Saxagliptin, polyethylene oxide and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with polyethylene oxide, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-18 capsule or tablet

| Ingredients | amount % (mg) |
|---|---|
| saxagliptin | 0,2 - 20 % |
| carbomer | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Saxagliptin, carbomer and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with carbomer, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-19 capsule or tablet

| Ingredients | amount % (mg) |
|---|---|
| saxagliptin | 0,2 - 20 % |
| polyethylene glycol | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1-3 |

The production of the formulation is realized like this. Saxagliptin, polyethylene glycol and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with polyethylene glycol, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-20 capsule or tablet

| Ingredients | amount % (mg) |
|---|---|
| saxagliptin | 0,2 - 20 % |
| Sodium alginate and/or alginic acid | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Saxagliptin, sodium alginate and/or alginic acid and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with sodium alginate and/or alginic acid, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-21 capsule or tablet

| Ingredients | amount % (mg) |
|---|---|
| saxagliptin | 0,2 - 20 % |
| polyvinyl alcohol | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Saxagliptin, polyvinyl alcohol and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with polyvinyl alcohol, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-22 capsule or tablet

| Ingredients | amount % (mg) |
|---|---|
| saxagliptin | 0,2 - 20 % |
| poloxamer | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Saxagliptin, poloxamer and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, saxagliptin is mixed with poloxamer, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-23 Capsule or Tablet

| **Ingredients** | **amount % (mg)** |
|---|---|
| sitagliptin | 5 - 70 % |
| polycarbophil | 0,1 - 30 % |
| silicified microcrystalline cellulose | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized like this. Sitagliptin, polycarbophil and silicified microcrystalline cellulose are mixed and granulated in roller compactor or briquette pressed. Then, the mixture is granulated by sieving with an appropriate screen. To the obtained granule, croscarmellose sodium and colloidal silicone dioxide are added after being sieved and they are mixed. Afterwards, magnesium stearate is added after being sieved and mixed for a short time. The tablet is compressed with the obtained powder ready for compression or the capsule is filled. The coating process is realized with a coating material having humidity barrier such as Opadry AMB/ Kollicoat IR.

In another production method, sitagliptin is mixed with polycarbophil and silicified microcrystalline cellulose. Half of the magnesium stearate is added to this mixture by sieving. After mixing for a short time, the mixture is mangled from the roller compactor or briquette pressed and granulated by sieving. To the obtained granules, croscarmellose sodium and colloidal silicone dioxide are added and mixed. The rest of the magnesium stearate is added to the this mixture by sieving and after mixing for a short time the tablet compressing and capsule filling processes are realized. The coating process is realized with a coating material having humidity barrier such as Opadry AMB/ Kollicoat IR.

### Example-24 capsule or tablet

| **Ingredients** | **amount % (mg)** |
|---|---|
| sitagliptin | 5 - 70 % |
| polycarbophil | 0,1 - 30 % |
| vinyl pyrrolidone-vinyl acetate copolymer or low viscosity hydroxy propyl cellulose | 2 - 40 % |
| silicified microcrystalline cellulose or dibasic calcium phosphate anhydrate | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized as below. Sitagliptin, polycarbophil, vinyl pyrrolidone-vinyl acetate copolymer or low viscosity hydroxy propyl cellulose and silicified microcrystalline cellulose or dibasic calcium phosphate anhydrate are mixed. The mixture is granulated in roller compactor or briquette pressed and granulated by sieving through the suitable sieve. To the obtained granule, croscarmellose sodium and colloidal silicone dioxide are added by sieving and they are mixed. Afterwards, magnesium stearate is added by sieving and mixed for a short time. The tablet is compressed with the obtained powder ready for compression or the capsule is filled. The coating process is realized with a coating material having humidity barrier such as Opadry AMB/ Kollicoat IR.

In another production method, sitagliptin is mixed with polycarbophyl, low viscosity hydroxy propyl cellulose and dibasic calcium phosphate anhydrate. Half of the magnesium stearate is sieved and added to the powder mixture and after mixing for a short time, the mixture is mangled from the roller compactor or slug pressed and granulated by sieving. Croscarmellose sodium and colloidal silicone dioxide are added to the obtained granules and mixed. The rest of the magnesium stearate is added to the this mixture by sieving and mixed for a short time and the tablet pressing process and capsule filling are realized. The coating process is realized with a coating material having humidity barrier such as Opadry AMB/ Kollicoat IR.

### Example-25 capsule or tablet

| Ingredients | amount % (mg) |
|---|---|
| sitagliptin | 5 - 70 % |
| low viscosity hydroxy propyl cellulose | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, low viscosity hydroxy propyl cellulose and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with polycarbophil, low viscosity hydroxy propyl methyl cellulose, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-26 Capsule or Tablet

| **Ingredients** | **amount % (mg)** |
|---|---|
| sitagliptin | 5 - 70 % |
| methyl cellulose | 0,1 - 30 % |
| silicified microcrystalline cellulose | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized like this. Sitagliptin, methyl cellulose and silicified microcrystalline cellulose are mixed and granulated in roller compactor or briquette pressed. Then, the mixture is granulated by sieving with an appropriate screen. To the obtained granule, croscarmellose sodium and colloidal silicone dioxide are added after being sieved and they are mixed. Afterwards, magnesium stearate is added after being sieved and mixed for a short time. The tablet is compressed with the obtained powder ready for compression or the capsule is filled. The coating process is realized with a coating material having humidity barrier such as Opadry AMB/ Kollicoat IR.

In another production method, sitagliptin is mixed with methyl cellulose and silicified microcrystalline cellulose. Half of the magnesium stearate is added to this mixture by sieving. After mixing for a short time, the mixture is mangled from the roller compactor or briquette pressed and granulated by sieving. To the obtained granules, croscarmellose sodium and colloidal silicone dioxide are added and mixed. The rest of the magnesium stearate is added to the this mixture by sieving and after mixing for a short time the tablet compressing and capsule filling processes are realized. The coating process is realized with a coating material having humidity barrier such as Opadry AMB/ Kollicoat IR.

### Example-27 capsule or tablet

| **Ingredients** | **amount % (mg)** |
|---|---|
| sitagliptin | 5 - 70 % |
| polycarbophil | 0,1 - 30 % |
| methyl cellulose | 2 - 40 % |
| silicified microcrystalline cellulose or dibasic calcium phosphate anhydrate | 5 - 90 % |
| croscarmellose sodium | 0,25 - 20 % |
| colloidal silicone dioxide | 0,1 - 1 % |
| magnesium stearate | 0,1 - 3 % |

The production of the formulation is realized as below. Sitagliptin, polycarbophil, methyl cellulose and silicified microcrystalline cellulose or dibasic calcium phosphate anhydrate are mixed. The mixture is granulated in roller compactor or briquette pressed and granulated by sieving through the suitable sieve. To the obtained granule, croscarmellose sodium and colloidal silicone dioxide are added by sieving and they are mixed. Afterwards, magnesium stearate is added by sieving and mixed for a short time. The tablet is compressed with the obtained powder ready for compression or the capsule is filled. The coating process is realized with a coating material having humidity barrier such as Opadry AMB/ Kollicoat IR.

In another production method, sitagliptin is mixed with polycarbophyl, methyl cellulose and dibasic calcium phosphate anhydrate. Half of the magnesium stearate is sieved and added to the powder mixture and after mixing for a short time, the mixture is mangled from the roller compactor or slug pressed and granulated by sieving. Croscarmellose sodium and colloidal silicone dioxide are added to the obtained granules and mixed. The rest of the magnesium stearate is added to the this mixture by sieving and mixed for a short time and the tablet pressing process and capsule filling are realized. The coating process is realized with a coating material having humidity barrier such as Opadry AMB/ Kollicoat IR.

### Example-28 capsule or tablet

| Ingredients | amount % (mg) |
|---|---|
| sitagliptin | 5 - 70 % |
| polyethylene oxide | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, polyethylene oxide and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with polyethylene oxide, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-29 capsule or tablet

| Ingredients | amount % (mg) |
|---|---|
| sitagliptin | 5 - 70 % |
| carbomer | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, carbomer and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with carbomer, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-30 capsule or tablet

| Ingredients | amount % (mg) |
|---|---|
| sitagliptin | 5 - 70 % |
| polyethylene glycol | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, polyethylene glycol and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with polyethylene glycol, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-31 capsule or tablet

| Ingredients | amount % (mg) |
|---|---|
| sitagliptin | 5 - 70 % |
| Sodium alginate and/or alginic acid | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, sodium alginate and/or alginic acid and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with sodium alginate and/or alginic acid, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-32 capsule or tablet

| Ingredients | amount % (mg) |
|---|---|
| sitagliptin | 5 - 70 % |
| polyvinyl alcohol | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, polyvinyl alcohol and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with polyvinyl alcohol, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

### Example-33 capsule or tablet

| Ingredients | amount % (mg) |
|---|---|
| sitagliptin | 5 - 70 % |
| poloxamer | 2 - 40 % |
| silicified microcrystalline cellulose | 5 - 90 % |
| dibasic calcium phosphate anhydrate | 5 - 90 % |
| croscarmellose sodium | 0,25 - 20 % |
| colloidal silicone dioxide | % 0,1 - 1 |
| magnesium stearate | % 0,1 - 3 |

The production of the formulation is realized like this. Sitagliptin, poloxamer and silicified microcrystalline cellulose and dibasic calcium phosphate anhydrate are mixed. The mixture is granulated in roller compactor or briquette pressed and granulated by sieving through a suitable sieve. Croscarmellose sodium and colloidal silicone dioxide are added to the obtained granule by sieving and they are mixed. Afterwards, magnesium stearate is added by sieving and mixed for a short time. The tablet is compressed with the obtained powder ready for compression or the capsule filling is realized. The coating process is realized with a coating material having humidity barrier such as Opadry AMB/ Kollicoat IR.

In another production method, sitagliptin is mixed with poloxamer, silicified microcrystalline cellulose and dibasic calcium phosphate anhydrate. Half of the magnesium stearate is sieved and added to the powder mixture and after mixing for a short time, the mixture is mangled from the roller compactor or briquette pressed and granulated by sieving. Croscarmellose sodium and colloidal silicone dioxide are added to the obtained granules and mixed. The rest of the magnesium stearate is added by sieving and mixed for a short time and the tablet pressing process and capsule filling are realized. The coating process is realized with a coating material having humidity barrier such as Opadry AMB/ Kollicoat IR.

Surprisingly, with the invention performed, a stable formulation with high solubility and high dissolution rate is obtained. The said formulation comprises DPP-IV inhibitor with at least one dry granulation excipient (binder). The preferred DPP-IV inhibitor is vildagliptin. Another preferred DPP-IV inhibitor is saxagliptin. One of the preferred DPP-IV inhibitor is sitagliptin. With the procedure applied, the need for using any liquid solvent including water is eliminated while a uniform formulation content is obtained. Thus, a formulation with high stability is obtained and the energy needed for the process is minimized. As heat is not used in the procedure, possible stability problems of the active ingredient are eliminated. Content uniformity is provided by means of granules obtained by briquette compression or compression between rollers. Besides that, possible fluidity problems experienced during production process are prevented. In the formulation, the ratio interval of vildagliptin to polycarbophil is 1 - 100, preferably 6 - 80, and more preferably 10 - 50. The desired dissolution rate and solubility values occur within these intervals. The characteristics of polycarbophil such as dry binding structure and having a high compatibility have a positive effect on the stability of the formulation. The tablet hardness preferred in the production of the formulation is 20-300 N, preferably 30 - 250 N, and more preferably 50 - 100 N. Preferably, the compression force for the dry granulation of the formulation is 1-25 KN, preferably 3 - 18 KN, and more preferably 5 - 10 KN. Owing to the preferred intervals of the said compression forces, ideal tablet and granule hardness is obtained with the dry granulation process. At the same time, the dispersal profile of the obtained tablet or granule occurs at a desired level. The average particle size (d50) of the granules is 25- 1250 µm, preferably 75- 800 µm and more preferably 100- 600 µm. Magnesium stearate is separately used in the inner and outer phases of the formulation. The usage of magnesium stearate at the inner phase of the formulation prevents sticking to the strips located in the rollers during compression between them, while the usage of magnesium stearate at the outer phase of the formulation prevents sticking to the puncher during tablet compression. A high fluidity is provided in the formulation by the preferred silicified microcrystalline cellulose.

The term granule also covers the particle or pellet meanings.

Opadry AMB is a coating substance comprising polyvinyl alcohol and it has the characteristics of air and moister barrier. Kollicoat IR contains polyvinyl alcohol - polyethylene glycol graft copolymer and it has the characteristics of air and moisture barrier.

The pharmaceutical compositions of the present invention may additionally contain one or more pharmaceutically acceptable excipients. The pharmaceutically acceptable and suitable excipients contain but may not be limited to filling substances, glidants, lubricants, disintegrants, surface active agents and the like and the mixtures of them.

The present invention is used for preventing and treating diabetes in mammals, especially in humans.

The said dry granulation excipients are used as binders in the formulation. The said excipients are selected among the ones with small particle size and having a large surface area. For example, celluloses and derivatives thereof with low viscosity and small particle size are preferred. Especially N-vinyl pyrrolidone-vinyl acetate copolymer and hydroxypropyl cellulose are selected with small particle sizes.
Besides lactose, the other excipients used may be spray dried lactose and spray dried lactose and starch mixtures. Besides MCC, spray dried microcrystalline cellulose, dibasic calcium phosphate anhydrate mixtures and spray dried calcium carbonate + microcrystalline cellulose mixtures may also be preferred. Microcrystalline cellulose with a small particle size is preferred.
It is also possible to use the additional excipients mentioned below in the formulation.

Suitable disintegrants contain but not limited to alginic acid and alginates, ion-exchanging resins, magnesium aluminum silica, sodium dodecil sulfate, sodium carboxymethyl cellulose, croscarmellose sodium, cross-linked PVP, carboxymethyl cellulose calcium, docusate sodium, guar gum, low-substituted HPC, polyacryline potassium, poloxamer, povidone, sodium alginate, sodium glycine carbonate and sodium lauryl sulfate and the like and the mixtures of them.

Suitable filling materials are sugars, mannitol, sorbitol, sucrose, inorganic salts, calcium salts, polysaccarides, dextrose, dicalcium phosphate, sodium chloride, dextrates, lactitol, maltodextrine, sucrose-maltodextrine mixtures, xylitol, trehalose, heavy magnesium carbonate, galena IQ(isomalt), LudiFlash (mannitol, crospovidone and polyvinyl acetate), Pharmaburst, Panexcea (microcrystalline cellulose, HPMC and crospovidone), F-Melt.

Suitable binding agents contain, but are not limited to polyvinylpyrrolidone, polyethylene glycol, polyvinyl alcohol, cellulose derivatives such as hydroxypropyl methyl cellulose, carboxy methyl cellulose, methyl cellulose, starch, collagen, gelatine, sugars, aluminium hydroxide, polyvinylalcohol, carrageenan, inorganic substances such as bentonite, laponite; surfactants such as setostearyl alcohol, polyoxyethylene-alkyl ethers; starch mucilage, acacia mucilage, polydextrose, polyethylene oxide, pullulan, guar gum, xanthene gum and the like and their mixtures.

Suitable lubricants comprise but not limited to magnesium stearate, calcium stearate, sodium stearyl fumarate, sodium lauryl sulfate, silica, talk, calcium stearate, polyethylene glycol, paraffin, sodium stearyl fumarate, sodium lauryl sulfate, magnesium lauryl sulfate, fumaric acid, glyceryl palmitostearate, hydrogenated vegetable oils, zinc stearate.stearic acid and the like and the mixtures of them.

Suitable glidants comprise but not limited to colloidal silicone dioxide, talk, aluminium silicate and the like and mixtures of them.

Suitable coating agents contain, but are not limited to semisyhthetic polymers such as poloxamer, polyacrylamide, hydroxypropyl cellulose, ethyl cellulose, hydroxy propyl methyl cellulose and methyl cellulose, polymethacrylates (such as Eudragit E and RD varieties), polyvinyl acetate, cellulose acetate phthalate, ethylene vinyl acetate, methyl aminoethyl methacrylate, neutral methacrylic acid esters, polyactide, polyactide co-glycolide, low molecular weight polyethylene/poly-isobutylene, polyanhydrates and poly(ortho-ester)s, diethyl aminoethyl methacrylate, Kollicoat smartseal 30D and the like and their mixtures.

The orally disintegrating compositions of the invention may also comprise sweeteners and aromatic agents in order to increase the patient compliance.

Suitable coloring agents comprise but not limited to food, drug and cosmetic (FD&C) dyes (e.g. FD&C blue, FD&C green, FD&C red, FD&C yellow, FD&C lacquer), ponso, indigo drug & cosmetic (D&C) blue, indigotine FD&C blue, carmoisine indigotine (indigo Carmine); iron oxide (e.g iron oxide red, yellow, black), quinoline yellow, flame, carmine, carmoisine, sunset yellow, ponso and the like and the mixtures of them.

## Claims

1. A solid pharmaceutical dosage formulation obtained with briquette pressing or compression between rollers and **characterized in that** it comprises DPP-IV inhibitor with at least one binding dry granulation excipient which is polycarbophil.

2. The pharmaceutical formulation according to claim 1 wherein the said formulation does not contain liquid solvent.

3. The formulation according to any one of the preceding claims wherein the said DPP-IV inhibitor is at least one of the substances selected from the group comprising vildagliptin, saxagliptin and sitagliptin.

4. The pharmaceutical formulation according to any one of the preceding claims, wherein the said DPP-IV inhibitor is vildagliptin.

5. The pharmaceutical formulation according to any one of the preceding claims wherein as a binding dry granulation excipient, it further comprise at least one substance or a mixture of substances selected among calcium carbonate, microcrystalline cellulose, silicified microcrystalline cellulose, hydroxy propyl cellulose, hydroxy propyl methyl cellulose, ethyl cellulose, methyl cellulose, polyvinyl alcohol, polyethylene glycol, carbomer, methacrylic acid copolymer, amino methacrylate copolymer, beeswax, mixture of solid maltodextrin-corn syrup, corn starch, hydrolyzed gelatine, polyvinyl acetate, polyvinyl pyrrolidone, polyvinyl pyrrolidone-vinyl acetate copolymer, sugars, glucose syrup, natural gums (glisirhiza gum, guar gum, tragagant gum), collagen, agar, alginate, sodium alginate, carrageenans, hyaluronic acid, pectin, poloxamer, polyacrylamide, polyvinyl alcohol, aluminium hydroxide, bentonite, laponit, setostearyl alcohol, polyoxyethylene-alkyl ethers, acacia mucilage, polydextrose, polyethylene oxide.

6. The pharmaceutical formulation according to any one of the preceding claims wherein the ratio interval of vildagliptin to polycarbophil is 1 - 100, preferably 6 - 80, and more preferably 10 - 50.

7. The pharmaceutical formulation according to any one of the preceding claims wherein further comprising at least one or more excipients.

8. The pharmaceutical formulation according to any one of the preceding claims wherein the said excipient comprises at least one of the substances among diluents, disintegrants, glidants and lubricants or a mixture of them in a suitable amount.

9. The pharmaceutical formulation according to any one of the preceding claims wherein it comprises at least one of the substances or a mixture of them as the said diluent, selected among lactose, starch, calcium carbonate, microcrystalline cellulose, silicified microcrystalline cellulose, mannitol, sorbitol, sucrose, dextrose, dextrates, lactitol, maltodextrine, xylitol, trehalose, dibasic calcium phosphate anhydrate and dibasic calcium phosphate dihydrate.

10. The pharmaceutical formulation according to any one of the preceding claims wherein the said disintegrant comprises at least one of the substances selected among croscarmellose sodium and sodium starch glycolate or a mixture of them in suitable amounts.

11. The pharmaceutical formulation according to any one of the preceding claims wherein the said glidant is colloidal silicone dioxide.

12. The pharmaceutical formulation according to any one of the preceding claims wherein as a lubricant it preferably comprise at least one of the substances selected among polyethylene glycol or magnesium stearate or it contains a mixture of them in suitable amounts.

13. The pharmaceutical formulation according to any one of the preceding claims wherein at least a part of the said lubricant or lubricant mixture is used in the inner granule phase and the rest of it is used in the outer granule phase.

14. The pharmaceutical formulation according to any one of the preceding claims wherein the average particle size (d50) of the granules obtained by briquette compression or by pressing between cylinders is between 25- 1250 µm, preferably between 75- 800 µm and more preferably between 100- 600 µm.

15. The pharmaceutical formulation according to any one of the preceding claims wherein the said formulation is in the form of tablet or capsule.

16. The pharmaceutical formulation according to any one of the preceding claims wherein the tablet hardness is 20-300 N, preferably 30 - 250 N, more preferably 50 - 100 N.

17. The pharmaceutical formulation according to any one of the preceding claims wherein the compression force of the dry granulation process is 1-25 KN, preferably 3- 18 KN, more preferably 5- 10 KN.

18. The pharmaceutical formulation according to any one of the preceding claims consisting of,
a. 5-60 % vildagliptin or a pharmaceutically acceptable salt of it by weight,
b. 0.1-30 % polycarbophil by weight
c. 5-90 % silicified microcrystalline cellulose by weight
d. 0.25-20 % croscarmellose sodium by weight
e. 0.1-1 % colloidal silicone dioxide by weight
f. 0.1-3 % magnesium stearate by weight

19. The pharmaceutical formulation according to any one of the preceding claims consisting of,
a. 5-60 % vildagliptin or a pharmaceutically acceptable salt of it by weight,
b. 0.1-30 % polycarbophil by weight
c. 2-40 % vinyl pyrrolidone-vinyl acetate copolymer or low viscosity hydroxy propyl cellulose by weight
d. 5-90 % silicified microcrystalline cellulose by weight
e. 0.25-20 % croscarmellose sodium by weight
f. 0.1-1 % colloidal silicone dioxide by weight
g. 0.1-3 % magnesium stearate by weight

20. A method for preparing a pharmaceutical formulation according to any one of the preceding claims, comprising the steps below:
a. Mixing vildagliptin, polycarbophil and silicified microcrystalline cellulose,
b. granulating then the mixture in a roller compactor or making briquette press and granulating it by sieving with a suitable sieve,
c. adding croscarmellose sodium and colloidal silicone dioxide to the obtained granule by sieving and mixing them,
d. afterwards, adding magnesium stearate to this mixture by sieving and mixing for a short time,
e. tablet compressing with the obtained powder ready for compression or filling it into a capsule,
f. coating the tablet with polyvinyl alcohol or polyvinyl alcohol polyethylene glycol graft copolymer.

21. A method for preparing a pharmaceutical formulation according to any one of the preceding claims, comprising the steps below:
a. Mixing vildagliptin, polycarbophil, vinyl pyrrolidone-vinyl acetate copolymer or low viscosity hydroxy propyl cellulose and silicified microcrystalline cellulose,
b. then, granulating the mixture in the roller compactor or making briquette press and granulating it by sieving with a suitable sieve,
c. adding croscarmellose sodium and colloidal silicone dioxide to the obtained granule by sieving and mixing them,
d. afterwards, adding magnesium stearate to this mixture by sieving and mixing for a short time,
e. tablet compressing with the obtained powder ready for compression or filling it into a capsule,
f. coating the tablet with polyvinyl alcohol or polyvinyl alcohol polyethylene glycol graft copolymer.

22. A method for preparing a pharmaceutical formulation according to any one of the preceding claims, comprising the steps below:
a. mixing vildagliptin, binder and diluent,
b. afterwards, adding at least a fragment of magnesium stearate into this mixture and mixing them,
c. then, granulating the mixture in the roller compactor or making briquette press and granulating it by sieving with a suitable sieve,
d. adding croscarmellose sodium and colloidal silicone dioxide to the obtained granule by sieving and mixing them,
e. afterwards, adding rest of the magnesium stearate to this mixture by sieving and mixing for a short time,
f. tablet compressing with the obtained powder ready for compression or filling it into a capsule,
g. coating the tablet with polyvinyl alcohol or polyvinyl alcohol polyethylene glycol graft copolymer.

23. The pharmaceutical formulation according to any of the preceding claims for use in preventing or treating diabetes mellitus in mammalians and particularly in humans.

## Patentansprüche

1. Pharmazeutische Formulierung in fester Darreichungsform, hergestellt durch Brikettierpressung oder Walzenpressung und **dadurch gekennzeichnet, dass** sie DPP-4-Inhibitoren mit mindestens einem Trockengranulierungs-Binde-Hilfsstoff umfasst, bei dem es sich um Polycarbophil handelt.

2. Pharmazeutische Formulierung nach Anspruch 1, bei der die Formulierung kein flüssiges Lösungsmittel enthält.

3. Pharmazeutische Formulierung nach einem der vorhergehenden Ansprüche, bei der der DPP-4-Inhibitor mindestens eine der Substanzen ausgewählt aus der Gruppe mit Vildagliptin, Saxagliptin und Sitagliptin ist.

4. Pharmazeutische Formulierung nach einem der vorhergehenden Ansprüche, bei der der DPP-4-Inhibitor Vildagliptin ist.

5. Pharmazeutische Formulierung nach einem der vorhergehenden Ansprüche, bei der sie als Trockengranulierungs-Binde-Hilfsstoff des Weiteren mindestens eine Substanz oder ein Gemisch von Substanzen umfasst, ausgewählt aus Calciumcarbonat, mikrokristalliner Cellulose, verkieselter mikrokristalliner Cellulose, Hydroxypropylcellulose, Hydroxypropylmethylcellulose, Ethylcellulose, Methylcellulose, Polyvinylalkohol, Polyethylenglykol, Carbomer, Methacrylsäurecopolymer, Aminomethacrylatcopolymer, Bienenwachs, einem Gemisch aus festem Maltrodextrin und Maissirup, Maisstärke, hydrolysierter Gelatine, Polyvinylacetat, Polyvinylpyrrolidon, Polyvinylpyrrolidon/Vinylacetat-Copolymer, Zuckern, Glukosesyrup, Pflanzengummis (Süßholz-Gummi, Guar-Gummi, Traganth-Gummi), Kollagen, Agar, Alginat, Natriumalginat, Carrageenane, Hyaluronsäure, Pektin, Poloxamer, Polyacrylamid, Polyvinylalkohol, Aluminiumhydroxid, Bentonit, Laponit, Cetylstearylalkohol, Polyoxyethylenalkylether, Akazienschleim, Polydextrose, Polyethylenoxid.

6. Pharmazeutische Formulierung nach einem der vorhergehenden Ansprüche, bei der das Verhältnis von Vildagliptin zu Polycarbophil im Bereich 1 - 100, vorzugsweise 6 - 80, und noch bevorzugter 10 - 50 liegt.

7. Pharmazeutische Formulierung nach einem der vorhergehenden Ansprüche, weiterhin umfassend mindestens einen oder mehrere Hilfsstoff(e).

8. Pharmazeutische Formulierung nach einem der vorhergehenden Ansprüche, bei der der Hilfsstoff mindestens eine der Substanzen Verdünnungsmittel, Fließreguliermittel und Schmiermittel oder ein Gemisch daraus in einer geeigneten Menge umfasst.

9. Pharmazeutische Formulierung nach einem der vorhergehenden Ansprüche, bei der sie mindestens eine der Substanzen oder ein Gemisch daraus als das Verdünnungsmittel umfasst, ausgewählt aus Lactose, Stärke, Calciumcarbonat, mikrokristalliner Cellulose, verkieselter mikrokristalliner Cellulose, Mannitol, Sorbitol, Saccharose, Dextrose, Dextrate, Lactitol, Maltodextrin, Xylitol, Trehalose, dibasisches Calciumphosphat-Anhydrat und dibasisches Calciumphosphat-Dihydrat.

10. Pharmazeutische Formulierung nach einem der vorhergehenden Ansprüche, bei der das Sprengmittel mindestens eine der Substanzen ausgewählt aus Croscarmellose-Natrium und Natriumstärkeglykolat oder ein Gemisch daraus in geeigneten Mengen umfasst.

11. Pharmazeutische Formulierung nach einem der vorhergehenden Ansprüche, bei der das Fließreguliermittel kolloidales Siliziumdioxid ist.

12. Pharmazeutische Formulierung nach einem der vorhergehenden Ansprüche, bei der sie als Schmiermittel vorzugsweise eine der Substanzen ausgewählt aus Polyethylenglykol oder Magnesiumstearat umfasst oder sie ein Gemisch daraus in geeigneten Mengen enthält.

13. Pharmazeutische Formulierung nach einem der vorhergehenden Ansprüche, bei der zumindest ein Teil des Schmiermittels bzw. des Schmiermittelgemisches in der inneren granulären Phase und der Rest in der äußeren granulären Phase verwendet werden.

14. Pharmazeutische Formulierung nach einem der vorhergehenden Ansprüche, bei der die durchschnittliche Teilchengröße (d50) des durch Brikettierung oder durch Walzenpressung erhaltenen Granulats zwischen 25 und 1250 µm, vorzugsweise zwischen 75 und 800 µm, und noch bevorzugter zwischen 100 und 600 µm beträgt.

15. Pharmazeutische Formulierung nach einem der vorhergehenden Ansprüche, bei der die Formulierung in Tabletten- oder Kapselform vorliegt.

16. Pharmazeutische Formulierung nach einem der vorhergehenden Ansprüche, bei der die Tablettenhärte 20 bis 300 N, vorzugsweise 30 bis 250 N, noch bevorzugter 50 bis 100 N beträgt.

17. Pharmazeutische Formulierung nach einem der vorhergehenden Ansprüche, bei der die Komprimierungskraft des Trockengranulierprozesses 1 bis 25 kN, vorzugsweise 3 bis 18 kN, noch bevorzugter 5 bis 10 kN beträgt.

18. Pharmazeutische Formulierung nach einem der vorhergehenden Ansprüche, bestehend aus:
a. 5-60 Gew.-% Vildagliptin oder einem pharmazeutisch akzeptablen Salz davon,
b. 0,1-30 Gew.-% Polycarbophil,
c. 5-90 Gew.-% verkieselter mikrokristalliner Cellulose,
d. 0,25-20 Gew.-% Croscarmellose-Natrium,
e. 0,1-1 Gew.-% kolloidalem Siliziumdioxid,
f. 0,1-3 Gew.-% Magnesiumstearat.

19. Pharmazeutische Formulierung nach einem der vorhergehenden Ansprüche, bestehend aus:
a. 5-60 Gew.-% Vildagliptin oder einem pharmazeutisch akzeptablen Salz davon,
b. 0,1-30 Gew.-% Polycarbophil,
c. 2-40 Gew.-% Vinylpyrrolidon/Vinylacetat-Copolymer oder niedrig viskose Hydroxypropylcellulose,
d. 5-90 Gew.-% verkieselter mikrokristalliner Cellulose,
e. 0,25-20 Gew.-% Croscarmellose-Natrium,
f. 0,1-1 Gew.-% kolloidalem Siliziumdioxid,
g. 0,1-3 Gew.-% Magnesiumstearat.

20. Verfahren zur Herstellung einer pharmazeutischen Formulierung nach einem der vorhergehenden Ansprüche, mit folgenden nachstehenden Schritten:
a. Mischen von Vildagliptin, Polycarbophil und verkieselter mikrokristalliner Cellulose,
b. anschließendes Granulieren des Gemisches in einer Walzenpresse oder einer Brikettierpresse und Granulieren durch Sieben mit einem geeigneten Sieb,
c. Zugabe von Croscarmellose-Natrium und kolloidalem Siliziumdioxid zum erhaltenen Granulat durch Sieben und Mischen,
d. anschließende Zugabe von Magnesiumstearat zu diesem Gemisch durch Sieben und Mischen über eine kurze Zeitdauer,
e. Pressen des kompressionsbereiten erhaltenen Pulvers zu Tabletten oder Befüllen einer Kapsel mit dem Pulver,
f. Überziehen der Tablette mit Polyvinylalkohol oder Polyvinylalkohol-Polyethylenglykol-Propf-Copolymer.

21. Verfahren zur Herstellung einer pharmazeutischen Formulierung nach einem der vorhergehenden Ansprüche, mit den nachstehend aufgeführten Schritten:
a. Mischen von Vildagliptin, Polycarbophil, Vinylpyrrolidon/Vinylacetat-Copolymer oder niedrig viskoser Hydroxypropylcellulose und verkieselter mikrokristalliner Cellulose,
b. anschließendes Granulieren des Gemisches in einer Walzenpresse oder einer Brikettierpresse und Granulieren durch Sieben mit einem geeigneten Sieb,
c. Zugabe von Croscarmellose-Natrium und kolloidalem Siliziumdioxid zum erhaltenen Granulat durch Sieben und Mischen,
d. anschließende Zugabe von Magnesiumstearat zu diesem Gemisch durch Sieben und Mischen über eine kurze Zeitdauer,
e. Pressen des kompressionsbereiten erhaltenen Pulvers zu Tabletten oder Befüllen einer Kapsel mit dem Pulver,
f. Überziehen der Tablette mit Polyvinylalkohol oder Polyvinylalkohol-Polyethylenglykol-Propf-Copolymer.

22. Verfahren zur Herstellung einer pharmazeutischen Formulierung gemäß einem der vorhergehenden Ansprüche, mit folgenden nachstehenden Schritten:
a. Mischen von Vildagliptin, Bindemittel und Verdünnungsmittel,
b. danach Zugabe mindestens eines Teils des Magnesiumstearats zu diesem Gemisch und Mischen,
c. anschließendes Granulieren des Gemisches in einer Walzenpresse oder einer Brikettierpresse und Granulieren durch Sieben mit einem geeigneten Sieb,
d. Zugabe von Croscarmellose-Natrium und kolloidalem Siliziumdioxid zum erhaltenen Granulat durch Sieben und Mischen dieser,
e. danach Zugabe des restlichen Magnesiumstearats zu diesem Gemisch durch Sieben und Mischen über eine kurze Zeitdauer,
f. Pressen des erhaltenen kompressionsbereiten Pulvers oder Befüllen einer Kapsel mit dem Pulver,
g. Überziehen der Tablette mit Polyvinylalkohol oder Polyvinylalkohol-Polyethylenglykol-Propf-Copolymer.

23. Pharmazeutische Formulierung nach einem der vorhergehenden Ansprüche zur Verwendung bei der Vorbeugung oder Behandlung von Diabetes mellitus bei Säugern und insbesondere Menschen.

## Revendications

1. Formulation posologique pharmaceutique solide obtenue par compression de briquette ou compression entre des rouleaux et **caractérisée en ce qu'**elle comprend un inhibiteur de la DPP-IV avec au moins un excipient de granulation à sec de liaison qui est polycarbophile.

2. Formulation pharmaceutique selon la revendication 1, dans laquelle ladite formulation ne contient pas de solvant liquide.

3. Formulation pharmaceutique selon l'une quelconque des revendications précédentes, dans laquelle ledit inhibiteur de la DPP-IV est au moins l'une des substances sélectionnées dans le groupe comprenant la vildagliptine, la saxagliptine et la sitagliptine.

4. Formulation pharmaceutique selon l'une quelconque des revendications précédentes, dans laquelle ledit inhibiteur de la DPP-IV est la vildagliptine.

5. Formulation pharmaceutique selon l'une quelconque des revendications précédentes, dans laquelle en tant qu'excipient de granulation à sec de liaison, elle comprend en outre au moins une substance ou un mélange de substances sélectionnées parmi le carbonate de calcium, la cellulose microcristalline, la cellulose microcristalline silicifiée, l'hydroxypropylcellulose, l'hydroxypropylméthylcellulose, l'éthylcellulose, la méthylcellulose, l'alcool polyvinylique, le polyéthylène glycol, un carbomère, un copolymère d'acide méthacrylique, un copolymère d'aminométhacrylate, la cire d'abeille, un mélange solide de maltodextrine-sirop de maïs, l'amidon de maïs, la gélatine hydrolysée, l'acétate de polyvinyle, la polyvinylpyrrolidone, un copolymère de polyvinylpyrrolidone et d'acétate de vinyle, les sucres, le sirop de glucose, les gommes naturelles (gomme de réglisse, gomme de guar, gomme adragante), le collagène, l'agar, l'alginate, l'alginate de sodium, les carraghénanes, l'acide hyaluronique, la pectine, le poloxamère, la polyacrylamide, l'alcool polyvinylique, l'hydroxyde d'aluminium, la bentonite, la laponite, l'alcool cétostéarylique, les éthers alkyliques de polyoxyéthylène, le mucilage d'acacia, la polydextrose, l'oxide de polyéthylène.

6. Formulation pharmaceutique selon l'une quelconque des revendications précédentes, dans laquelle le rapport de l'écart entre la vildagliptine et le polycarbophile est de 1-100, de préférence de 6-80 et plus préférablement de 10-50.

7. Formulation pharmaceutique selon l'une quelconque des revendications précédentes, comprenant en outre au moins un ou plusieurs excipients.

8. Formulation pharmaceutique selon l'une quelconque des revendications précédentes, dans laquelle ledit excipient comprend au moins l'une des substances parmi les diluants, les déliants, les agents d'écoulement et les lubrifiants ou un mélange de celles-ci dans une quantité appropriée.

9. Formulation pharmaceutique selon l'une quelconque des revendications précédentes, dans laquelle elle comprend au moins l'une des substances ou un mélange de celles-ci en tant que ledit diluant, sélectionnées parmi le lactose, l'amidon, le carbonate de calcium, la cellulose microcristalline, la cellulose microcristalline silicifiée, le mannitol, le sorbitol, le sucrose, le dextrose, les dextrates, le lactitol, la maltodextrine, le xylitol, le tréhalose, le phosphate de calcium dibasique anhydre et le phosphate de calcium dibasique dihydraté.

10. Formulation pharmaceutique selon l'une quelconque des revendications précédentes, dans laquelle ledit déliant comprend au moins l'une des substances sélectionnées parmi la croscarmellose sodique et le glycolate d'amidon sodique ou un mélange de ceux-ci dans des quantités appropriées.

11. Formulation pharmaceutique selon l'une quelconque des revendications précédentes, dans laquelle ledit agent d'écoulement est le dioxyde de silice colloïdale.

12. Formulation pharmaceutique selon l'une quelconque des revendications précédentes, dans laquelle en tant que lubrifiant elle comprend de préférence au moins l'une des substances sélectionnées parmi le polyéthylène glycol ou le stéarate de magnésium ou elle contient un mélange de ceux-ci dans des quantités appropriées.

13. Formulation pharmaceutique selon l'une quelconque des revendications précédentes, dans laquelle au moins une partie dudit lubrifiant ou mélange de lubrifiants est utilisée dans la phase granulaire interne et le reste est utilisé dans la phase granulaire externe.

14. Formulation pharmaceutique selon l'une quelconque des revendications précédentes, dans laquelle la taille de particules moyenne (d50) des granules obtenus par compression de briquette ou par compression entre des rouleaux se situe entre 25-1250 µm, de préférence entre 75-800 µm et plus préférablement entre 100-600 µm.

15. Formulation pharmaceutique selon l'une quelconque des revendications précédentes, dans laquelle ladite formulation se présente sous la forme d'un comprimé ou d'une gélule.

16. Formulation pharmaceutique selon l'une quelconque des revendications précédentes, dans laquelle la dureté du comprimé est de 20-300 N, de préférence de 30-250 N, plus préférablement de 50-100 N.

17. Formulation pharmaceutique selon l'une quelconque des revendications précédentes, dans laquelle la force de compression du processus de granulation à sec est de 1-25 kN, de préférence de 3-18 kN, plus préférablement de 5-10 kN.

18. Formulation pharmaceutique selon l'une quelconque des revendications précédentes, constituée par
a. 5-60 % de vildagliptine ou d'un sel pharmaceutiquement acceptable de celle-ci en poids,
b. 0,1-30 % de polycarbophile en poids,
c. 5-90 % de cellulose microcristalline silicifiée en poids,
d. 0,25-20 % de croscarmellose sodique en poids,
e. 0,1-1 % de dioxyde de silice colloïdale en poids,
f. 0,1-3 % de stéarate de magnésium en poids.

19. Formulation pharmaceutique selon l'une quelconque des revendications précédentes, constituée par
a. 5-60 % de vildagliptine ou d'un sel pharmaceutiquement acceptable de celle-ci en poids,
b. 0,1-30 % de polycarbophile en poids,
c. 2-40 % de copolymère de vinylpyrrolidone-acétate de vinyle ou d'hydroxypropylcellulose de faible viscosité en poids,
d. 5-90 % de cellulose microcristalline silicifiée en poids,
e. 0,25-20 % de croscarmellose sodique en poids,
f. 0,1-1 % de dioxyde de silice colloïdale en poids,
g. 0,1-3 % de stéarate de magnésium en poids.

20. Procédé de préparation d'une formulation pharmaceutique selon l'une quelconque des revendications précédentes, comprenant les étapes ci-dessous :
a. mélanger la vildagliptine, le polycarbophile et la cellulose microcristalline silicifiée,
b. granuler ensuite le mélange dans un compacteur à rouleaux ou réaliser une compression de briquette et granuler par tamisage avec un tamis approprié,
c. ajouter la croscarmellose sodique et le dioxyde de silice colloïdale à la granule obtenue par tamisage et les mélanger,
d. ensuite, ajouter le stéarate de magnésium à ce mélange par tamisage et mélanger pendant un court moment,
e. former le comprimé par compression avec la poudre obtenue prête pour la compression ou garnir une gélule avec celle-ci,
f. enrober le comprimé avec de l'alcool polyvinylique ou un copolymère greffé d'alcool polyvinylique et de polyéthylène glycol.

21. Procédé de préparation d'une formulation pharmaceutique selon l'une quelconque des revendications précédentes, comprenant les étapes ci-dessous :
a. mélanger la vildagliptine, le polycarbophile, le copolymère de vinylpyrrolidone et d'acétate de vinyle ou l'hydroxypropylcellulose de faible viscosité et la cellulose microcristalline silicifiée,
b. ensuite, granuler le mélange dans un compacteur à rouleaux ou réaliser une compression de briquette et granuler par tamisage avec un tamis approprié,
c. ajouter la croscarmellose sodique et le dioxyde de silice colloïdale à la granule obtenue par tamisage et les mélanger,
d. ensuite, ajouter le stéarate de magnésium à ce mélange par tamisage et mélanger pendant un court moment,
e. former le comprimé par compression avec la poudre obtenue prête pour la compression ou garnir une gélule avec celle-ci,
f. enrober le comprimé avec de l'alcool polyvinylique ou un copolymère greffé d'alcool polyvinylique et de polyéthylène glycol.

22. Procédé de préparation d'une formulation pharmaceutique selon l'une quelconque des revendications précédentes, comprenant les étapes ci-dessous :
a. mélanger la vildagliptine, le liant et le diluant,
b. ensuite, ajouter un fragment de stéarate de magnésium dans ce mélange et les mélanger,
c. ensuite, granuler le mélange dans le compacteur à rouleaux ou réaliser une compression de briquette et granuler par tamisage avec un tamis approprié,
d. ajouter la croscarmellose sodique et le dioxyde de silice colloïdale à la granule obtenue par tamisage et les mélanger,
e. ensuite, ajouter le reste de stéarate de magnésium à ce mélange par tamisage et mélanger pendant un court moment,
f. former le comprimé par compression avec la poudre obtenue prête pour la compression ou garnir une gélule avec celle-ci,
g. enrober le comprimé avec de l'alcool polyvinylique ou un copolymère greffé d'alcool polyvinylique et de polyéthylène glycol.

23. Formulation pharmaceutique selon l'une quelconque des revendications précédentes en vue d'une utilisation dans la prévention ou le traitement du diabète sucré chez les mammifères et en particulier chez l'homme.
